# EUROPÄISCHE PATENTSCHRIFT

(11) **EP 3 992 516 B1**
(45) Veröffentlichungstag und Bekanntmachung des Hinweises auf die Patenterteilung: **23.08.2023**
(21) Anmeldenummer: 21204462.2
(22) Anmeldetag: 25.10.2021
(51) Int. Cl.: A61M 39/14, A61M 39/16, A61M 39/18, A61M 39/10, F16L 23/04

(54) **SYSTEM UND VERFAHREN ZUR BEREITSTELLUNG EINER STERILEN FLUIDVERBINDUNG ZWISCHEN EINEM ERSTEN FLUIDKANAL UND EINEM ZWEITEN FLUIDKANAL**
SYSTEM AND METHOD FOR PROVIDING A STERILE FLUID CONNECTION BETWEEN A FIRST FLUID CHANNEL AND A SECOND FLUID CHANNEL
SYSTÈME ET PROCÉDÉ DE FOURNITURE D'UNE LIAISON FLUIDIQUE STÉRILE ENTRE UN PREMIER CANAL DE FLUIDE ET UN SECOND CANAL DE FLUIDE

(30) Priorität: 27.10.2020 AT 509262020
(43) Veröffentlichungstag der Anmeldung: 04.05.2022
(73) Patentinhaber: Zeta GmbH, 8501 Lieboch (AT)
(72) Erfinder: Pittermann, Birgit, 8504 Preding (AT); Pöschl, Georg, 8020 Graz (AT); Trummer, Erwin, 8083 St. Stefan im Rosental (AT); Weingartshofer, Reinhard, 8561 Hitzendorf (AT)
(74) Vertreter: Röggla, Harald

(56) Entgegenhaltungen:
- EP-A1- 3 225 895
- WO-A1-98/50105
- US-A- 3 865 411
- US-A1- 2015 028 586

## Beschreibung

Die Erfindung betrifft ein System zur Bereitstellung einer sterilen Fluidverbindung zwischen einem ersten Fluidkanal und einem zweiten Fluidkanal gemäß dem Oberbegriff des Anspruchs 1.

Die Erfindung betrifft des Weiteren ein Verfahren zur Bereitstellung einer sterilen Fluidverbindung mit einem erfindungsgemäßen System.

In den Bereichen der Biopharmazie und der Lebensmittelindustrie werden hohe Standards in Bezug auf Produktion, Lagerung und Transport von Produkten angelegt. Gesetzliche Vorgaben in diesen Bereichen regulieren unter anderem die Sterilität von Produktionsprozessen, Verarbeitungsprozessen und Anwendungen der Produkte sowie die Durchführung von Produktprüfungen. Im Rahmen der Produktion besteht somit aufgrund von Qualitätssicherungsanforderungen und den gesetzlichen Vorgaben die Notwendigkeit Produkte und Produktbestandteile in einem möglichst sterilen Umfeld zu halten.

In der Regel befinden sich flüssige Produkte der Biopharmazie oder der Lebensmittelindustrie im Produktionsprozess in großen aseptischen Materialbehältern in welchen diese transportiert, zwischengelagert oder verarbeitet werden. Im Zuge des Produktionsprozesses, von Analysen in Laboren, oder einfach bei der Umlagerung von derartigen Produkten von einem Materialbehälter in einen anderen Materialbehälter, oder von einem Materialbehälter zu einer Verarbeitungsvorrichtung oder einer Analysevorrichtung besteht die Notwendigkeit Fluidverbindungen zum Transport der Produkte bereitzustellen. Die Bereitstellung einer Fluidverbindung ist jedoch immer mit dem Risiko verbunden, Fremdkörper oder Keime in die Produkte einzubringen. Eine große Herausforderung auf dem Gebiet der Biopharmazie und der Lebensmittelindustrie besteht somit darin, die Sterilität der Produkte bei der Bereitstellung von Fluidverbindungen zu wahren.

Systeme zur Bereitstellung von sterilen Fluidverbindungen gemäß dem Stand der Technik umfassen in der Regel zwei Kopplungselemente, welche jeweils im Bereich der Mündung jeweils eines Fluidkanals bereitgestellt sind. Die Mündungen sind zusätzlich mit einer abziehbaren Folie verschlossen. In der Regel ist die Folie mittels eines Klebers auf die Mündung aufgeklebt, oder die Folie ist auf die Mündung aufgepresst, und stellt einen sterilen Verschluss des Fluidkanals bereit. Die Folie kann auch zusätzlich verschweißt oder versiegelt sein. Ein typisches System gemäß dem Stand der Technik ist aus der US 8,454,059 B2 bekannt. Um eine Kopplung zu erreichen werden in diesem System zwei Kopplungselemente verbunden, wodurch die beiden Mündungen der Fluidkanäle im Wesentlichen deckungsgleich angeordnet werden. Diese Verbindung wird mittels Einhakelementen erreicht, welche an den Kopplungselementen bereitgestellt sind. Danach werden die Folien, welche die beiden Mündungen verschließen, gleichzeitig abgezogen, wodurch die Fluidverbindung hergestellt wird. Anschließend wird bei der US 8,454,059 B2 ein aufschraubbares Sicherungselement oder Verbindungselement verwendet, um die Fluidverbindung gegenüber mechanischen Belastungen zu sichern.

Ein Nachteil des Standes der Technik besteht darin, dass im Zuge der Entfernung der Folien von den Mündungen die Gefahr besteht, dass Fremdstoffe in die Fluidkanäle gelangen und somit die, durch die Fluidkanäle fließenden Produkte verunreinigen. Des Weiteren weisen bekannte Systeme den Nachteil auf, dass diese nur eine einmalige, Single-Use Verwendung ermöglichen, und eine Trennung von einmal verbundenen Fluidkanälen nicht angedacht ist. Hierdurch weisen bekannte Systeme eine verringerte Wirtschaftlichkeit auf, und tragen durch ihre nur einmalige Verwendbarkeit zur Umweltverschmutzung bei. Darüber hinaus weisen bekannte Systeme Einschränkungen hinsichtlich der maximalen Druckbelastung im verbundenen Zustand, unter welchen die Sterilität der hergestellten Verbindung aufrechterhalten werden kann, sowie beispielsweise der Temperaturbereiche auf in welchen diese zuverlässig einsetzbar sind. Zusätzlich ist die mechanische Stabilität im verbundenen Zustand eingeschränkt, wodurch es aufgrund hoher Manipulationskräfte, wie hohe auftretende Momente durch befestigte Schläuche, etc. zu Leckagen im Verbindungsbereich kommen kann. Die mangelnde Temperaturbeständigkeit ist insbesondere nachteilig bei der inline Verwendung derartiger Systeme im eingebauten Zustand bei Sterilisationsprozessen unter Anwendung von überhitztem- oder Sattdampf. Auch in einem ausgebauten Zustand sind somit Anwendungen in Autoklaven in der Regel nicht möglich.

Die US 3,865,411 A offenbart ein Verbindungsstück zum Verbinden von zwei Leitungen sowie ein Verfahren für das Zustandekommen einer einfachen, sterilen und betriebssicheren Verbindung zwischen den zwei Leitungen, sodass keine Kontamination der internen Leitungsoberflächen durch Materie von außen stattfinden kann.

Die WO 98/50105 A1 offenbart ein Verbindungssystem für eine Fluidverbindung mit einem ersten und einem zweiten Verbindungsstück, sowie einer Aufnahme für das erste oder das zweite Verbindungsstück.

Die EP 3 225 895 A1 offenbart ein Verbindungsstück zum Herstellen einer Fluidverbindung.

Die US 2015/028586 A1 offenbart eine Vorrichtung zur Herstellung einer sterilen Verbindung.

Es ist die Aufgabe der vorliegenden Erfindung, ein System zur Bereitstellung einer sterilen Fluidverbindung zwischen einem ersten Fluidkanal und einem zweiten Fluidkanal sowie ein Verfahren zur Bereitstellung einer sterilen Fluidverbindung zu schaffen, welche die oben angeführten Nachteile vermeiden.

Erfindungsgemäß wird die vorliegende Aufgabe durch die Bereitstellung eines Systems zur Bereitstellung einer sterilen Fluidverbindung zwischen einem ersten Fluidkanal und einem zweiten Fluidkanal mit den Merkmalen von Anspruch 1 gelöst.

Des Weiteren wird die vorliegende Aufgabe durch die Bereitstellung eines Verfahrens zur Bereitstellung einer sterilen Fluidverbindung mit den Merkmalen von Anspruch 13 gelöst.

Das erfindungsgemäße System zur Bereitstellung einer sterilen Fluidverbindung zwischen einem ersten Fluidkanal und einem zweiten Fluidkanal umfasst ein, an einer Mündung des ersten Fluidkanals angeordnetes erstes Kopplungselement, und ein, an einer Mündung des zweiten Fluidkanals angeordnetes zweites Kopplungselement. Dass erste Kopplungselement und das zweite Kopplungselement umfassen jeweils eine, die Mündung des jeweiligen Fluidkanals verschließende, und von der Mündung abziehbare Folie. Erfindungsgemäß umfasst das System zudem ein von dem ersten Kopplungselement und dem zweiten Kopplungselement gesondertes Verbindungselement, welches dazu ausgebildet ist das erste Kopplungselement und das zweite Kopplungselement im Bereich der Mündungen der Fluidkanäle zumindest abschnittsweise aufzunehmen, und die mit den Folien verschlossenen Mündungen der Fluidkanäle aneinanderzupressen. Das Verbindungselement weist zudem eine, im Wesentlichen in der Ebene der Mündungen der Fluidkanäle angeordnete Ausnehmung zur Durchführung der Folien durch das Verbindungselement auf.

Das erfindungsgemäße Verfahren zur Bereitstellung einer sterilen Fluidverbindung mit dem erfindungsgemäßen System umfasst die Schritte:
- Anordnen der Mündung des ersten Fluidkanals im Wesentlichen deckungsgleich mit der Mündung des zweiten Fluidkanals;
- Zumindest abschnittsweises Aufnehmen des ersten Kopplungselements und des zweiten Kopplungselements im Bereich der Mündungen der Fluidkanäle durch das Verbindungselement;
- Durchführen der die Mündungen der Fluidkanäle verschließenden, und von der jeweiligen Mündung abziehbaren Folien durch die Ausnehmung in dem Verbindungselement;
- Herstellen der sterilen Fluidverbindung durch gleichzeitiges Abziehen der Folien durch die Ausnehmung des Verbindungselements hindurch.

Durch die erfindungsgemäße Ausführung des Verfahrens beziehungsweise des Systems zur Bereitstellung einer sterilen Fluidverbindung mit einem von dem ersten Kopplungselement und dem zweiten Kopplungselement getrennten Verbindungselement, sowie der in dem Verbindungselement vorgesehenen Ausnehmung zur Durchführung der Folien durch das Verbindungselement wird der Vorteil erreicht, dass das Verbindungselement das erste Kopplungselement und das zweite Kopplungselement im Bereich der Mündungen der Fluidkanäle aufnehmen kann, bevor die Folien durch die Ausnehmung in dem Verbindungselement abgezogen werden. Hierdurch wird eine sichere Verbindung der Fluidkanäle, sowie ein Anpressdruck der beiden Mündungen aneinander beziehungsweise der beiden Kopplungselemente aneinander erreicht, bevor die Folien entfernt werden. Hierdurch wird effektiv verhindert, dass Fremdkörper und/oder Keime in die Fluidverbindung im Zuge des Abziehens der Folien gelangen, wodurch die Sterilität der Verbindung sichergestellt wird. Zusätzlich wird die Fluidverbindung bereits vor dem Abziehen der Folien mechanisch stabilisiert, und auch gegen große mechanische Belastungen gesichert, wodurch die Sicherheit der Verbindung zusätzlich erhöht wird. Die im Verbindungselement vorgesehene Ausnehmung stellt eine Führung der Folien bereit, wodurch ein gleichmäßiges und gerades Abziehen der Folien sichergestellt wird. Der Anpressdruck bei dieser ersten Kopplung ist derart gewählt, dass die Folien noch durch die Ausnehmung des Verbindungselements gezogen werden können, ohne zu reißen. Der Anpressdruck, der durch das Verbindungselement generiert wird, ist in diesem Zustand somit groß genug um die Sterilität der Verbindung zu gewährleisten. Durch die Trennung des Verbindungselements von dem ersten Kopplungselement und dem zweiten Kopplungselement wird zudem der Vorteil erreicht, dass die Fluidverbindung durch einfaches Abnehmen des Verbindungselements getrennt werden kann. Nach erfolgter Reinigung, Sterilisierung und dem Anbringen neuer Folien steht das System für eine erneute Benutzung bereit. Hierdurch wird die Wirtschaftlichkeit des Systems erhöht, und Abfall reduziert.

Das Verbindungselement des erfindungsgemäßen Systems ist als Überwurfklemme ausgebildet. Diese Ausgestaltung des Verbindungselements stellt den Vorteil bereit, dass eine hohe Vorspannkraft bei der Aufnahme des ersten Kopplungselements und des zweiten Kopplungselements im Bereich der Mündungen der Fluidkanäle erreicht werden kann. Hierdurch wird die mechanische Stabilität der Fluidverbindung erhöht, und das Risiko einer Kontamination der Fluidverbindung mit Keimen beim Abziehen der Folien reduziert.

Gemäß einer bevorzugten Ausführungsform des erfindungsgemäßen Systems weisen das erste Kopplungselement und das zweite Kopplungselement im Bereich der Mündungen eine kegelstumpfförmige Aufweitung auf. Durch die kegelstumpfförmige Aufweitung wird eine große Angriffsfläche bereitgestellt, an welche das Verbindungselement im Zuge der Aufnahme der Kopplungselemente angreifen kann.

Die Überwurfklemme ist vorzugsweise dazu ausgebildet, die kegelstumpfförmigen Aufweitungen der Kopplungselemente zumindest abschnittsweise aufzunehmen, wobei die Überwurfklemme ein Klemmprofil aufweist, welches auf die kegelstumpfförmigen Aufweitungen bei der Aufnahme der Kopplungselemente in die Überwurfklemme eine Klemmbelastung ausübt. Hierdurch wird die, durch die Überwurfklemme erzeugte Vorspannkraft weiter erhöht. Zudem wird durch diese Ausführung sichergestellt, dass immer dieselbe Klemmkraft angewandt wird, wodurch ein Reißen der Folien beim Abziehen vermieden wird.

Erfindungsgemäß weist die Überwurfklemme ein Unterteil, ein, mittels eines Scharniergelenks mit dem Unterteil verbundenes Oberteil, und ein, an einer dem Scharniergelenk im Wesentlichen gegenüberliegenden Seite angeordnetes Klemmelement zum Arretieren des Oberteils an dem Unterteil auf. Durch diese mehrteilige Ausführung der Überwurfklemme mit dem Klemmelement zur Arretierung kann die Überwurfklemme einfach und ohne großen Kraftaufwand sicher angebracht, und auch auf einfache Weise wieder gelöst werden. Vorzugsweise ist die Ausnehmung zur Durchführung der Folien in dem Oberteil der Überwurfklemme vorgesehen. Hierdurch wird das Durchführen der Folien durch das Verbindungselement erleichtert.

Erfindungsgemäß umfasst die Überwurfklemme ein mit dem Klemmelement gelenkig verbundenes Fixierelement, und ein im Bereich des Scharniergelenks angeordnetes Sperrelement. Das Fixierelement ist dazu ausgebildet, ausgehend von dem Klemmelement das Oberteil der Überwurfklemme zu überspannen, und das Sperrelement ist dazu ausgebildet das Fixierelement in einer das Oberteil der Überwurfklemme überspannenden Position zu Arretieren. Hierdurch wird eine Endfixierung der Überwurfklemme erreicht, welche nach dem Abziehen der Folien durch die Ausnehmung erfolgt. Hierdurch wird die mechanische Stabilität, die Klemmkraft und die Sicherheit der Verbindung weiter verbessert. Im Zustand dieser Endfixierung wird ein hoher Anpressdruck der Kopplungselemente aneinander erzeugt, wodurch der Betrieb der Verbindung mit einem hohen Betriebsdruck ermöglicht wird.

Das Unterteil, liegt vorzugsweise, wenn das erste Kopplungselement und das zweite Kopplungselement in der Überwurfklemme aufgenommenem sind, nur in einem zentralen Abschnitt an dem ersten Kopplungselement und dem zweiten Kopplungselement an. Hierdurch wird die Vorspannkraft der Überwurfklemme weiter erhöht und kann durch die Formgebung des Abschnitts 17 genau definiert werden.

Vorzugsweise weisen das erste Kopplungselement und das zweite Kopplungselement jeweils eine die Mündung umlaufende, von der Folie überdeckte Dichtung auf. Hierdurch wird eine zusätzliche Barriere gegen das Eindringen von Keimen in die Fluidverbindung bereitgestellt. Die Dichtungen sind unter den Folien und derart angeordnet, dass sie sowohl beim, als auch nach dem Abziehen der Folien eine sichere Abdichtung bereitstellen. Beim Abziehen der Folie entsteht zudem ein Spalt, welchen die Dichtungen unmittelbar verschließen, um die Sterilität der Verbindung durchgehend zu gewährleisten.

Gemäß der bevorzugten Ausführungsform des erfindungsgemäßen Systems umfasst das Fixierelement im Bereich des Klemmelements einen auf das Oberteil wirkenden Exzenter, wobei der Exzenter eine Druckbelastung auf das Oberteil auswirkt, wenn das Fixierelement das Oberteil der Überwurfklemme überspannt. Hierdurch wird die von der Überwurfklemme auf das erste Kopplungselement und das zweite Kopplungselement ausgewirkte Kraft, und der aus dieser Kraft resultierende Anpressdruck der Kopplungselemente aneinander beim Schließen des Fixierelements zusätzlich verstärkt.

Gemäß einer Ausführungsvariante des erfindungsgemäßen Systems umfassen das erste Kopplungselement und das zweite Kopplungselement miteinander koppelbare Lokalisationselemente, welche dazu ausgebildet sind, im Zuge einer Kopplung die Mündung des ersten Fluidkanals im Wesentlichen deckungsgleich mit der Mündung des zweiten Fluidkanals zu positionieren. Hierdurch wird eine optimale Positionierung des ersten Kopplungselements und des zweiten Kopplungselements sichergestellt.

Vorzugsweise umfassen die Folien jeweils eine, an einem Randbereich der Mündungen umgeschlagene, durch die Ausnehmung des Verbindungselements durchführbare Abziehlasche. Hierdurch wird das Abziehen der Folien erleichtert.

Vorzugsweise umfasst der Schritt des erfindungsgemäßen Verfahrens des Durchführens der die Mündungen der Fluidkanäle verschließenden, und von der jeweiligen Mündung abziehbaren Folien durch die Ausnehmung in dem Verbindungselement das Schließen des Oberteils der Überwurfklemme, das Durchführen der die Mündung des ersten Fluidkanals und des zweiten Fluidkanals verschließenden Folien durch die Ausnehmung in dem Oberteil der Überwurfklemme und das Arretieren des Oberteils an dem Unterteil mittels dem Klemmelement.

Das erfindungsgemäße Verfahren umfasst zudem die Schritte:
- Überspannen des Oberteils der Überwurfmutter mit dem Fixierelement;
- Arretieren des Fixierelement in der das Oberteil der Überwurfklemme überspannenden Position mittels des Sperrelements.
Hierdurch wird der Vorteil erreicht, hergestellte Fluidverbindung gegen unbeabsichtigtes Öffnen gesichert wird.

Vorteilhafte Ausgestaltungen des erfindungsgemäßen Systems und des erfindungsgemäßen Verfahrens, sowie alternativer Ausführungsvarianten werden in weiterer Folge anhand der Figuren näher erläutert.
Figur 1 zeigt das erfindungsgemäße System zur Bereitstellung einer sterilen Fluidverbindung zwischen einem ersten Fluidkanal und einem zweiten Fluidkanal in einem nicht zusammengesetzten Zustand.
Figur 2 zeigt das System gemäß Figur 1 in einem zusammengesetzten Zustand.
Figur 3 zeigt einen Längsschnitt durch das erfindungsgemäße System im zusammengesetzten Zustand.
Figur 4 zeigt einen Querschnitt durch das erfindungsgemäße System im zusammengesetzten Zustand.
Figur 5 zeigt einen Schnitt durch das erfindungsgemäße System gemäß Figur 4 entlang der Linie B-B.
Figur 6 zeigt eine alternative Ausführungsvariante einer Überwurfklemme des erfindungsgemäßen Systems.
Figur 7 zeigt die Überwurfklemme aus Figur 6 in einer Querschnittsdarstellung.
Figur 8 zeigt das erfindungsgemäße System in einer schematischen Darstellung mit einer zusätzlichen Vorrichtung zur Zugentlastung.

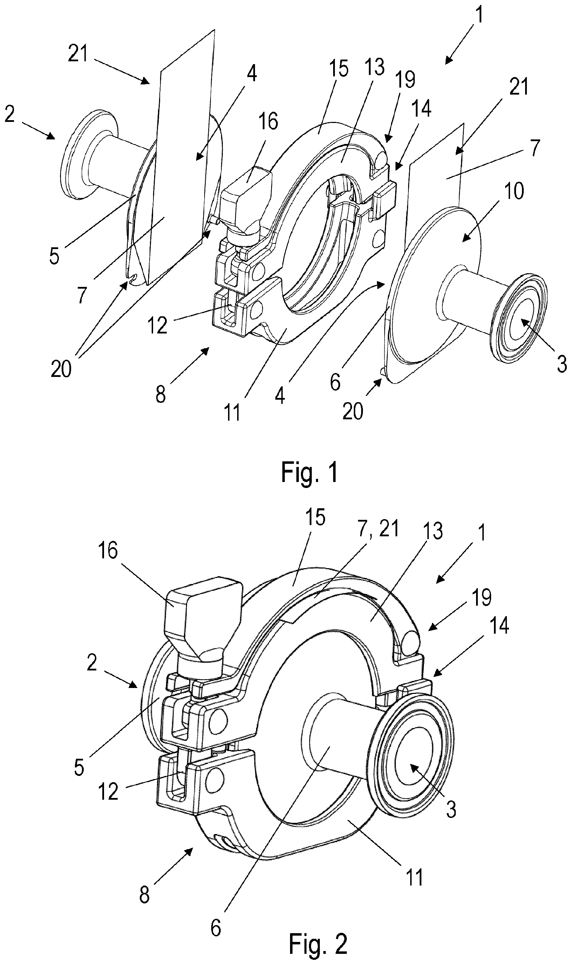

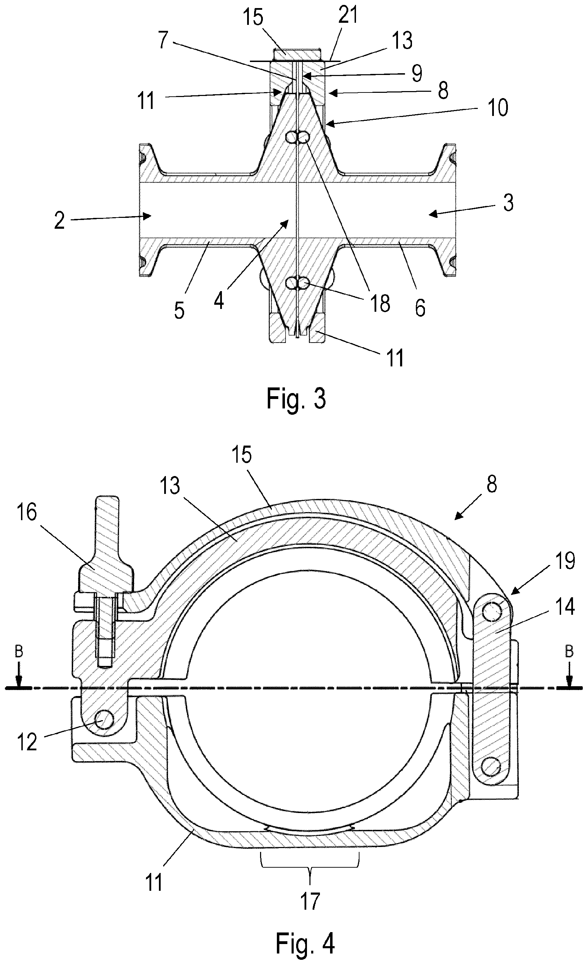

Figur 1 zeigt das erfindungsgemäße System 1 in einer perspektivischen Ansicht in einem nicht zusammengesetzten Zustand. Das erfindungsgemäße System 1 zur Bereitstellung einer sterilen Fluidverbindung zwischen einem ersten Fluidkanal 2 und einem zweiten Fluidkanal 3 umfasst ein, an einer Mündung 4 des ersten Fluidkanals 2 angeordnetes erstes Kopplungselement 5, und ein, an einer Mündung 4 des zweiten Fluidkanals 3 angeordnetes zweites Kopplungselement 6. Die Fluidkanäle 2 und 3 verlaufen hierbei vorzugsweise durch die Kopplungselemente 5 und 6 hindurch. Das erste Kopplungselement 5 und das zweite Kopplungselement 6 umfassen jeweils eine, die Mündung 4 des jeweiligen Fluidkanals 2 und 3 verschließende, und von der Mündung abziehbare Folie 7. Das erfindungsgemäße System 1 ist gekennzeichnet durch ein von dem ersten Kopplungselement 5 und dem zweiten Kopplungselement 6 gesondertes Verbindungselement 8. Das gesonderte Verbindungselement 8 ist dazu ausgebildet das erste Kopplungselement 5 und das zweite Kopplungselement 6 im Bereich der Mündungen 4 der Fluidkanäle 2 und 3 zumindest abschnittsweise aufzunehmen. Im Zuge der Aufnahme der Kopplungselemente 5 und 6 durch das Verbindungselement 8 werden die mit den Folien 7 verschlossenen Mündungen 4 der Fluidkanäle 2 und 3 aneinandergepresst. Diese Konfiguration des erfindungsgemäßen Systems 1 ist in Figur 2 und in Figur 3 ersichtlich, in welcher die Kopplungselemente 5 und 6 in dem Verbindungselement 8 aufgenommen dargestellt sind, wodurch das System 1 in einem zusammengesetzten Zustand vorliegt. Erfindungsgemäß weist das Verbindungselement 8 eine, im Wesentlichen in der Ebene der Mündung 4 des Fluidkanäle 2 und 3 angeordnete Ausnehmung 9 zur Durchführung der Folien 7 durch das Verbindungselement 8 auf. Die Ausnehmung 9 ist vorzugsweise schlitzförmig ausgebildet. Die Ausnehmung 9 ist in Figur 3 ersichtlich, in welcher das System 1 im zusammengesetzten Zustand mit dem Verbindungselement 8 in einem Längsschnitt dargestellt ist, wobei die Folien 7 in Figur 3 nach dem Durchtritt durch die Ausnehmung 9 in einem umgeklappten Zustand dargestellt sind. Die Ausnehmung 9 ermöglicht die Einhaltung eines immer gleichen Abziehwinkels der Folien 7.

Mit dem erfindungsgemäßen System 1 wird ein erfindungsgemäßes Verfahren zur Bereitstellung einer sterilen Fluidverbindung durchgeführt, welches folgende Schritte umfasst. Zuerst wird die Mündung 4 des ersten Fluidkanals 2 im Wesentlichen deckungsgleich mit der Mündung 4 des zweiten Fluidkanals 3 angeordnet. Danach wird das erste Kopplungselements 5 und des zweiten Kopplungselements 6 zumindest abschnittsweise im Bereich der Mündungen 4 der Fluidkanäle 2 und 3 durch das Verbindungselement 8 aufgenommen. In weiterer Folge werden die, die Mündungen 4 der Fluidkanäle 2 und 3 verschließenden, und von der jeweiligen Mündung 4 abziehbaren Folien 7 durch die Ausnehmung 9 in dem Verbindungselement 8 durchgeführt. Anschließend wird die sterile Fluidverbindung durch gleichzeitiges Abziehen der Folien 7 durch die Ausnehmung 9 des Verbindungselements 8 hindurch hergestellt. Im zuletzt genannten Verfahrensschritt zur Herstellung der sterilen Fluidverbindung werden die Folien 7 durch die Ausnehmung 9 in dem Verbindungselement 8 hindurch von den Mündungen 4 der Fluidkanäle 2 und 3 abgezogen.

Durch die erfindungsgemäße Ausführung des Verfahrens beziehungsweise des Systems 1 mit dem von dem ersten Kopplungselement 5 und dem zweiten Kopplungselement 6 getrennten Verbindungselement 8, sowie der in dem Verbindungselement 8 vorgesehenen Ausnehmung 9 zur Durchführung der Folien 7 durch das Verbindungselement 8 wird der Vorteil erreicht, dass das Verbindungselement 8 das erste Kopplungselement 5 und das zweite Kopplungselement 6 aufnehmen kann, bevor die Folien 7 durch die Ausnehmung 9 in dem Verbindungselement 8 abgezogen werden. Hierdurch wird gewährleistet, dass eine sichere, sterile, und mechanisch stabile Verbindung der Fluidkanäle 2 und 3 vorliegt, bevor die Folien 7 entfernt werden. Dies wird insbesondere auch dadurch erreicht, dass hierbei eine unsterile Seite der Folien die an der Mündung 4 anliegende, sterile beziehungsweise keimarme Innenseite der Fluidkanäle 3 und 4 nicht berührt. Hierdurch wird effektiv verhindert, dass Fremdkörper und/oder Keime in die Fluidverbindung im Zuge des Abziehens der Folien 7 gelangen, wodurch die Sterilität der Fluidverbindung sichergestellt wird. Zusätzlich wird die Fluidverbindung bereits vor dem Abziehen der Folien 7 mechanisch stabilisiert, und auch gegen große mechanische Belastungen gesichert, wodurch die Sicherheit der Fluidverbindung zusätzlich erhöht wird. Durch die getrennte Ausführung des Verbindungselements 8 von dem ersten Kopplungselement 5 und dem zweiten Kopplungselement 6 kann die Fluidverbindung durch einfaches Abnehmen des Verbindungselements von den Kopplungselementen 5 und 6 wieder getrennt werden kann. Nach erfolgter Reinigung, Sterilisierung und Anbringen neuer Folien 7 steht das System 1 für eine erneute Benutzung bereit. Hierdurch wird die Wirtschaftlichkeit des Systems 1 erhöht, und die Umweltverträglichkeit verbessert.

Vorzugsweise sind das Verbindungselement 8 und/oder die Kopplungselemente 5 und 6 aus Stahl gefertigt. Die Folien 7 sind vorzugsweise Verbundfolien aus handelsüblichen pharmakonformen und/oder lebensmittelechten Materialien, welche in einer Stanzmaschine vorkonfektioniert werden. Hierdurch kann das erfindungsgemäße System 1 in einem großen Druck- und Temperaturbereich verwendet werden. Vorzugsweise ist das System 1 für einen Druckbereich von 0 bis 10 bar Druck in der Fluidverbindung ausgelegt. Durch die erfindungsgemäße Ausführung können zudem das erste Kopplungselement 5 und das zweite Kopplungselement 6 der Verwendung an einen Schlauch angeschlossen und sanitisiert werden. Die vorkonfektionierten Folien 7 werden vorzugsweise mittels Druck und/oder Temperatur auf die Mündungen 4 aufgebracht. Durch die Hitze schmilzt das Siegelmaterial an der Folie 7 und kann nach dem Abkühlen rückstandsfrei abgezogen werden.

Wie in den Figuren ersichtlich, ist das Verbindungselement 8 vorzugsweise als Überwurfklemme ausgeführt. Die Ausführung als Überwurfklemme ermöglicht ein einfaches Anbringen und Entfernen des Verbindungselements 8, und stellt einen großen Anpressdruck des ersten Kopplungselements 5 an das zweite Kopplungselement 6 bereit.

Gemäß der in den Figuren dargestellten bevorzugten Ausführungsform des erfindungsgemäßen Systems 1 weisen das erste Kopplungselement 5 und das zweite Kopplungselement 6 im Bereich der Mündungen 4 eine kegelstumpfförmige Aufweitung 10 auf. Diese ist insbesondere in der Querschnittsdarstellung von Figur 3 ersichtlich. Durch die kegelstumpfförmige Aufweitung 10 wird eine große Angriffsfläche bereitgestellt, an welche das Verbindungselement 8 im Zuge der Aufnahme der Kopplungselemente 5 und 6 angreifen kann. Die Überwurfklemme ist gemäß der bevorzugten Ausführungsform dazu ausgebildet die kegelstumpfförmigen Aufweitungen 10 der Kopplungselemente 5 und 6 zumindest abschnittsweise aufzunehmen. Zudem weist die Überwurfklemme vorzugsweise ein Klemmprofil auf, welches auf die kegelstumpfförmigen Aufweitungen 10 bei der Aufnahme der Kopplungselemente 5 und 6 in die Überwurfklemme eine Klemmbelastung ausübt. Das Klemmprofil ist ebenfalls in Figur 3 ersichtlich. Hierdurch wird die durch die Überwurfklemme erzeugte Vorspannkraft noch weiter erhöht.

Wie in der Querschnittsdarstellung von Figur 4 ersichtlich, weist das als Überwurfklemme ausgeführte Verbindungelement 8 erfindungsgemäß ein Unterteil 11, ein, mittels eines Scharniergelenks 12 mit dem Unterteil 11 verbundenes Oberteil 13, und ein, an einer dem Scharniergelenk 12 im Wesentlichen gegenüberliegenden Seite angeordnetes Klemmelement 14 zum Arretieren des Oberteils 13 an dem Unterteil 11 auf. Durch diese mehrteilige und gelenkig verbundene Ausführung der Überwurfklemme mit dem Klemmelement 14 zur Arretierung kann die Überwurfklemme einfach und ohne großen Kraftaufwand sicher angebracht werden. Zudem wird hierdurch das erneute Lösen der Überwurfklemme auf einfache Weise ermöglicht. Vorzugsweise ist die Ausnehmung 9 zur Durchführung der Folien 7 in dem Oberteil 13 der Überwurfklemme vorgesehen, wie in Figur 3 ersichtlich. Hierdurch wird das Durchführen der Folien 7 durch das Verbindungselement 8 erleichtert.

Die Überwurfklemme weist erfindungsgemäß zudem ein mit dem Klemmelement 14 gelenkig verbundenes Fixierelement 15 auf, welches unter Anderem in Figur 4 dargestellt ist. Zudem ist in der Überwurfklemme ein im Bereich des Scharniergelenks 12 angeordnetes Sperrelement 16 umfasst, wobei das Fixierelement 15 dazu ausgebildet ist ausgehend von dem Klemmelement 14 das Oberteil 13 der Überwurfklemme zu überspannen, und das Sperrelement 16 dazu ausgebildet ist das Fixierelement 15 in einer das Oberteil 13 der Überwurfklemme überspannenden Position zu Arretieren. Hierdurch kann eine Fixierung der Überwurfklemme erreicht werden, welche nach dem Abziehen der Folien 7 durch die Ausnehmung 9 erfolgen kann. Hierdurch wird die mechanische Stabilität, die Klemmkraft und die Sicherheit der Verbindung weiter verbessert. Alternativ kann die Fixierung mittels dem Fixierelement 15 auch in einem Zwischenschritt vor dem Entfernen der Folien 7 erfolgen. Hierbei wird das Fixierelement 15 mittels des Sperrelements 16 arretiert, bevor die Folien 7 von den Mündungen 4 abgezogen wurden. Dies ist in Figur 2 und Figur 3 ersichtlich, wobei die Folien 7 durch die Ausnehmung 9 geführt sind, und zwischen dem Oberteil 13 und dem Fixierelement 15 hindurch in einen Außenbereich der Überwurfklemme verlaufen. Um die Folien 7 abzuziehen, und die Fluidverbindung herzustellen wird zuerst die Arretierung mittels des Sperrelements 16 gelöst, und anschließend das Fixierelement 15 von dem Oberteil 13 weggeschwenkt. Daraufhin können die Folien 7 durch die Ausnehmung 9 in dem Oberteil 13 hindurch abgezogen werden, ohne dass die Überwurfklemme vollständig geöffnet werden muss. Vorzugsweise ist das Sperrelement 16 als Flügelschraube ausgeführt. Alternative Ausführungsvarianten des Sperrelements sind dem Fachmann allgemein bekannt.

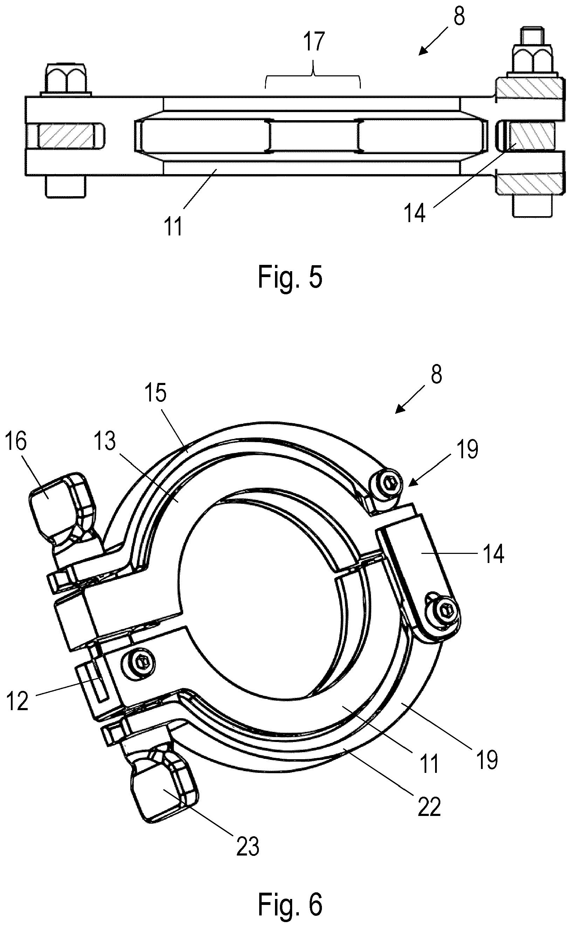

Vorzugsweise liegt das Unterteil 11, wie in Figur 4 ersichtlich, wenn das erste Kopplungselement 5 und das zweite Kopplungselement 6 in der Überwurfklemme aufgenommen sind, nur in einem zentralen Abschnitt 17 an dem ersten Kopplungselement 5 und dem zweiten Kopplungselement 6 an. Der zentrale Abschnitt 17 ist auch in Figur 5 ersichtlich, welche eine Schnittdarstellung von Figur 4 entlang der Linie B-B bildet. Hierdurch wird die Vorspannkraft der Überwurfklemme weiter erhöht. Besonders bevorzugt liegt der zentrale Abschnitt 17 an den kegelstumpfförmigen Aufweitungen 10 der Kopplungselemente 5 und 6 an. Hierdurch wird die Anpresskraft möglichst gleichförmig, und reproduzierbar entlang des zentralen Abschnitts 17 verteilt auf die Kopplungselemente 5 und 6 übertragen. Dies stellt den Vorteil bereit, dass kein zusätzliches Drehmoment auf die Kopplungselemente 5 und 6 im Zuge der Kopplung übertragen werden kann, wodurch ein Verkanten der Komponenten des erfindungsgemäßen Systems im Zuge der Herstellung der Verbindung verhindert wird.

Wie in Figur 3 ersichtlich weisen das erste Kopplungselement 5 und das zweite Kopplungselement 6 gemäß der bevorzugten Ausführungsform des erfindungsgemäßen Systems 1 jeweils eine die Mündung 4 umlaufende, von der Folie 7 überdeckte Dichtung 18 auf. Die Dichtung 18 bildet eine zusätzliche Barriere gegen das Eindringen von Keimen in den Fluidkanal, insbesondere beim Abziehen der Folien 7.

Zur zusätzlichen Erhöhung der Klemmkraft der Überwurfklemme umfasst das Fixierelement 15 gemäß der bevorzugten Ausführungsform des erfindungsgemäßen Systems 1 im Bereich des Klemmelements 14 einen auf das Oberteil 13 wirkenden Exzenter 19, wobei der Exzenter 19 eine Druckbelastung auf das Oberteil 13 auswirkt, wenn das Fixierelement 15 das Oberteil 13 der Überwurfklemme überspannt. Der Exzenter 19 ist beispielsweise in Figur 4 ersichtlich.

Das erste Kopplungselement 5 und das zweite Kopplungselement 6 weisen vorzugsweise miteinander koppelbare Lokalisationselemente 20 auf, welche in Figur 1 ersichtlich sind. Die Lokalisationselemente 20 sind dazu ausgebildet, im Zuge einer Kopplung die Mündung 4 des ersten Fluidkanals 5 im Wesentlichen deckungsgleich mit der Mündung 4 des zweiten Fluidkanals 6 zu positionieren. Hierdurch wird eine optimale Positionierung des ersten Kopplungselements 5 und des zweiten Kopplungselements 6 sichergestellt. Vorzugsweise sind die Lokalisationselemente 20, wie in Figur 1 dargestellt, als Nuten und Stifte ausgebildet, die jeweils ineinander eingreifen. Gemäß der bevorzugten Ausführungsvariante weist das erste Kopplungselement 5 eine Nut und einen Stift auf, und das zweite Kopplungselement 6 weist ebenfalls eine Nut und einen Stift auf, wobei der Stift des ersten Kopplungselements 5 in die Nut des zweiten Kopplungselements 6 eingreift und der Stift des zweiten Kopplungselements 6 in die Nut des ersten Kopplungselements 5 eingreift. Hierdurch wird eine einfache, reproduzierbare, und wieder lösbare Möglichkeit zur Ausrichtung der Kopplungselemente 5 und 6 aneinander geschaffen.

Vorzugsweise umfassen die Folien 7 jeweils eine, an einem Randbereich der Mündungen 4 umgeschlagene, durch die Ausnehmung 9 des Verbindungselements 8 durchführbare Abziehlasche 21. Die Abziehlasche 21 ist beispielsweise in Figur 1 ersichtlich. Hierdurch wird das Abziehen der Folien 7 von der Mündung 4 erleichtert.

Der Schritt des Durchführens der die Mündungen 4 der Fluidkanäle 2 und 3 verschließenden, und von der jeweiligen Mündung 2 abziehbaren Folien 7 durch die Ausnehmung 9 in dem Verbindungselement 8 in dem erfindungsgemäßen Verfahren zur Bereitstellung einer sterilen Fluidverbindung mit dem erfindungsgemäßen System 1 umfasst gemäß der bevorzugten Ausführungsform des Verfahrens einen weiteren Schritt. Dieser umfasst das Schließen des Oberteils 13 der Überwurfklemme, das Durchführen der die Mündung 4 des ersten Fluidkanals 2 und des zweiten Fluidkanals 3 verschließenden Folien 7 durch die Ausnehmung 9 in dem Oberteil 13 der Überwurfklemme und das Arretieren des Oberteils 13 an dem Unterteil 11 mittels dem Klemmelement 14.

Das erfindungsgemäße Verfahren umfasst zudem die Schritte des Überspannens des Oberteils 13 der Überwurfmutter mit dem Fixierelement 15 und des Arretierens des Fixierelements 15 in der das Oberteil 13 der Überwurfklemme überspannenden Position mittels des Sperrelements 16. Hierdurch wird der Vorteil erreicht, dass die hergestellte Fluidverbindung gegen unbeabsichtigtes Öffnen gesichert wird.

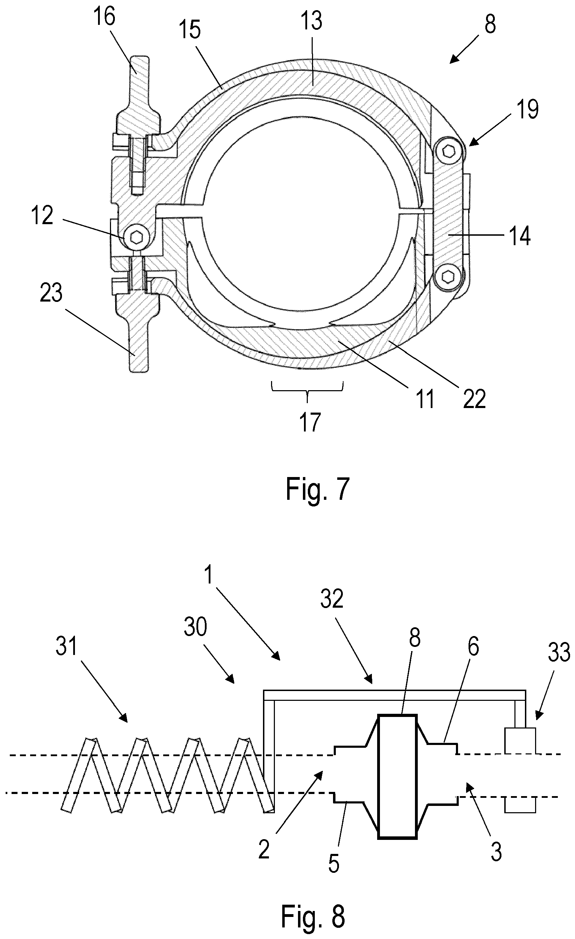

Figur 6 und Figur 7 zeigen eine alternative Ausführungsvariante des als Überwurfklemme ausgeführten Verbindungselements 8 des erfindungsgemäßen Systems 1, wobei gemäß dieser Ausführungsvariante die Überwurfklemme ein, dem Fixierelement 15 gegenüberliegendes, mit dem Klemmelement 14 gelenkig verbundenes zweites Fixierelement 22 umfasst. Zudem umfasst die Überwurfklemme ein dem Sperrelement 16 gegenüberliegendes zweites Sperrelement 23, wobei das zweite Fixierelement 22 dazu ausgebildet ist, ausgehend von dem Klemmelement 14 das Unterteil 11 der Überwurfklemme zu überspannen, und das zweite Sperrelement 23 dazu ausgebildet ist das zweite Fixierelement 22 in einer das Unterteil 11 der Überwurfklemme überspannenden Position zu fixieren. Hierdurch wird eine zusätzliche Sicherheit gegen unbeabsichtigtes Öffnen der Überwurfklemme bereitgestellt. Das zweite Sperrelement 23 ist vorzugsweise als Flügelschraube ausgeführt. Dem Fachmann sind mögliche zu Flügelschrauben alternative Ausführungen allgemein bekannt.

Figur 8 zeigt das erfindungsgemäße System 1 in einer alternativen Ausführungsvariante, welches eine zusätzliche Vorrichtung 30 zur Zugentlastung umfasst. Die Vorrichtung 30 umfasst eine Feder 31, einen mit der Feder verbundenen Bügel 32, und eine mit dem Bügel verbundene Klemme 33. Die Feder ist dazu ausgebildet im Betrieb der Vorrichtung 30 und im zusammengesetzten Zustand des Systems 1 den ersten Fluidkanal 2 innerhalb der Federwindungen, dem Verlauf der Feder 31 folgend aufzunehmen. Das erste Kopplungselement 5, das Verbindungselement 8 und das zweite Kopplungselement 6 werden im Betrieb der Vorrichtung 30 im Bereich des Bügels 32 angeordnet. Die Klemme 33 umgreift beziehungsweise klemmt im Betrieb den zweiten Fluidkanal 3. Alternativ kann die Klemme 33 auch den ersten Fluidkanal 2 umgreifen beziehungsweise klemmen, und der zweite Fluidkanal 3 ist in der Feder 31 angeordnet. Durch die Anordnung eines der Fluidkanäle 2 oder 3 innerhalb der Feder 31, und der starren Verbindung der Feder 31 über den Bügel 32 mit der Klemme 33 wird eine Zugentlastung des Verbindungselements 8 des Systems 1 erreicht, da das Gewicht des ersten Fluidkanals 2 und des zweiten Fluidkanals 3 durch die Vorrichtung 30 aufgenommen werden. Hierdurch werden die mechanische Stabilität und die Druckfestigkeit des Systems 1 verbessert.

## Patentansprüche

1. System (1) zur Bereitstellung einer sterilen Fluidverbindung zwischen einem ersten Fluidkanal (2) und einem zweiten Fluidkanal (3), umfassend ein, an einer Mündung (4) des ersten Fluidkanals (2) angeordnetes erstes Kopplungselement (5), und ein, an einer Mündung (4) des zweiten Fluidkanals (3) angeordnetes zweites Kopplungselement (6), wobei das erste Kopplungselement (5) und das zweite Kopplungselement (6) jeweils eine, die Mündung (4) des jeweiligen Fluidkanals (2, 3) verschließende, und von der Mündung (4) abziehbare Folie (7) umfassen,
wobei
das System (1) ein von dem ersten Kopplungselement (5) und dem zweiten Kopplungselement (6) gesondertes Verbindungselement (8) umfasst, welches dazu ausgebildet ist das erste Kopplungselement (5) und das zweite Kopplungselement (6) im Bereich der Mündungen (4) der Fluidkanäle (2, 3) zumindest abschnittsweise aufzunehmen, und die mit den Folien (7) verschlossenen Mündungen (4) der Fluidkanäle (2, 3) aneinanderzupressen, wobei das Verbindungselement (8) eine, im Wesentlichen in der Ebene der Mündungen (4) der Fluidkanäle (2, 3) angeordnete Ausnehmung (9) zur Durchführung der Folien (7) durch das Verbindungselement (8) aufweist, **dadurch gekennzeichnet, dass** das Verbindungselement (8) als Überwurfklemme ausgebildet ist, und die Überwurfklemme ein Unterteil (11), ein, mittels eines Scharniergelenks (12) mit dem Unterteil (11) verbundenes Oberteil (13), und ein, an einer dem Scharniergelenk (12) im Wesentlichen gegenüberliegenden Seite angeordnetes Klemmelement (14) zum Arretieren des Oberteils (13) an dem Unterteil (11) aufweist, wobei die Überwurfklemme ein mit dem Klemmelement (14) gelenkig verbundenes Fixierelement (15), und ein im Bereich des Scharniergelenks (12) angeordnetes Sperrelement (16) umfasst, wobei das Fixierelement (15) dazu ausgebildet ist ausgehend von dem Klemmelement (14) das Oberteil (13) der Überwurfklemme zu überspannen, und das Sperrelement (16) dazu ausgebildet ist das Fixierelement (15) in einer das Oberteil (13) der Überwurfklemme überspannenden Position zu arretieren.

2. System (1) gemäß Anspruch 1, **dadurch gekennzeichnet, dass** das erste Kopplungselement (5) und das zweite Kopplungselement (6) im Bereich der Mündungen (4) eine kegelstumpfförmige Aufweitung (10) aufweisen.

3. System (1) gemäß Anspruch 2, **dadurch gekennzeichnet, dass** die Überwurfklemme dazu ausgebildet ist die kegelstumpfförmigen Aufweitungen (10) der Kopplungselemente (5, 6) zumindest abschnittsweise aufzunehmen, wobei die Überwurfklemme ein Klemmprofil aufweist, welches auf die kegelstumpfförmigen Aufweitungen (10) bei der Aufnahme der Kopplungselemente (5, 6) in die Überwurfklemme eine Klemmbelastung ausübt.

4. System (1) gemäß Anspruch 1, **dadurch gekennzeichnet, dass** die Ausnehmung (9) zur Durchführung der Folien (7) in dem Oberteil (13) der Überwurfklemme vorgesehen ist.

5. System (1) gemäß einem der Ansprüche 1 oder 4, **dadurch gekennzeichnet, dass** das Unterteil (11), wenn das erste Kopplungselement (5) und das zweite Kopplungselement (6) in der Überwurfklemme aufgenommenem sind, nur in einem zentralen Abschnitt (17) an dem ersten Kopplungselement (5) und dem zweiten Kopplungselement (6) anliegt.

6. System (1) gemäß einem der Ansprüche 1 bis 5, **dadurch gekennzeichnet, dass** das erste Kopplungselement (5) und das zweite Kopplungselement (6) jeweils eine die Mündung (4) umlaufende, von der Folie (7) überdeckte Dichtung (18) aufweisen.

7. System (1) gemäß Anspruch 1, **dadurch gekennzeichnet, dass** das Fixierelement (15) im Bereich des Klemmelements (14) einen auf das Oberteil (13) wirkenden Exzenter (19) umfasst, wobei der Exzenter (19) eine Druckbelastung auf das Oberteil (13) auswirkt, wenn das Fixierelement (15) das Oberteil (13) der Überwurfklemme überspannt.

8. System (1) gemäß einem der Ansprüche 1 bis 7, **dadurch gekennzeichnet, dass** das erste Kopplungselement (5) und das zweite Kopplungselement (6) miteinander koppelbare Lokalisationselemente (20) umfassen, welche dazu ausgebildet sind, im Zuge einer Kopplung die Mündung (4) des ersten Fluidkanals (2) im Wesentlichen deckungsgleich mit der Mündung (4) des zweiten Fluidkanals (3) zu positionieren.

9. System (1) gemäß einem der Ansprüche 1 bis 8, **dadurch gekennzeichnet, dass** die Folien (7) jeweils eine, an einem Randbereich der Mündungen (4) umgeschlagene, durch die Ausnehmung (9) des Verbindungselements (8) durchführbare Abziehlasche (21) umfassen.

10. Verfahren zur Bereitstellung einer sterilen Fluidverbindung mit einem System (1) gemäß Anspruch 1, **gekennzeichnet durch** die Schritte:
- Anordnen der Mündung (4) des ersten Fluidkanals (2) im Wesentlichen deckungsgleich mit der Mündung (4) des zweiten Fluidkanals (3);
- Zumindest abschnittsweises Aufnehmen des ersten Kopplungselements (5) und des zweiten Kopplungselements (6) im Bereich der Mündungen (4) der Fluidkanäle (2, 3) durch das Verbindungselement (8);
- Durchführen der die Mündungen (4) der Fluidkanäle (2, 3) verschließenden, und von der jeweiligen Mündung (4) abziehbaren Folien (7) durch die Ausnehmung (9) in dem Verbindungselement (8);
- Herstellten der sterilen Fluidverbindung durch gleichzeitiges Abziehen der Folien (7) durch die Ausnehmung (9) des Verbindungselements (8) hindurch,
- Überspannen des Oberteils (13) der Überwurfklemme mit dem Fixierelement (15),
- Arretieren des Fixierelements (15) in der das Oberteil (13) der Überwurfklemme überspannenden Position mittels des Sperrelements (16).

11. Verfahren zur Bereitstellung einer sterilen Fluidverbindung gemäß Anspruch 10, mit einem System (1) gemäß den Ansprüchen 1 und 4 **dadurch gekennzeichnet, dass** der Schritt des Durchführens der die Mündungen (4) der Fluidkanäle (2, 3) verschließenden, und von der jeweiligen Mündung (4) abziehbaren Folien (7) durch die Ausnehmung (9) in dem Verbindungselement (8) das Schließen des Oberteils (13) der Überwurfklemme, das Durchführen der die Mündung (4) des ersten Fluidkanals (2) und des zweiten Fluidkanals (3) verschließenden Folien (7) durch die Ausnehmung (9) in dem Oberteil (13) der Überwurfklemme, und das Arretieren des Oberteils (13) an dem Unterteil (11) mittels dem Klemmelement (14) umfasst.

## Claims

1. A system (1) for providing a sterile fluid connection between a first fluid channel (2) and a second fluid channel (3), comprising a first coupling element (5) arranged at an orifice (4) of the first fluid channel (2) and a second coupling element (6) arranged at an orifice (4) of the second fluid channel (3), wherein the first coupling element (5) and the second coupling element (6) each comprise a foil (7) that closes the orifice (4) of the respective fluid channel (2, 3) and may be peeled off of the orifice (4),
wherein
the system (1) comprises a connecting element (8) separated from the first coupling element (5) and the second coupling element (6), which is configured to accommodate the first coupling element (5) and the second coupling element (6) in the region of the orifices (4) of the fluid channels (2, 3) at least in some sections and to press the orifices (4) of the fluid channels (2, 3), which are closed by the foils (7), against one another, wherein the connecting element (8) has a recess (9) arranged essentially in the plane of the orifices (4) of the fluid channels (2, 3) for passing the foils (7) through the connecting element (8), **characterized in that** the connecting element (8) is configured as a union clamp, and **in that** the union clamp has a bottom part (11), a top part (13) connected to the bottom part (11) by means of a hinged joint (12) and a clamping element (14) arranged on a side essentially opposite to the hinged joint (12) for locking the top part (13) at the bottom part (11), wherein the union clamp comprises a fixing element (15) connected to the clamping element (14) in an articulated way as well as a locking element (16) arranged in the region of the hinged joint (13), wherein the fixing element (15) is configured to span the top part (13) of the union clamp, starting from the clamping element (14), and wherein the locking element (16) is configured to lock the fixing element (15) in a position spanning the top part (13) of the union clamp.

2. A system (1) according to claim 1, **characterized in that** the first coupling element (5) and the second coupling element (6) have a frustoconical expansion (10) in the region of the orifices (4).

3. A system (1) according to the claims 2, **characterized in that** the union clamp is configured to accommodate the frustoconical expansions (10) of the coupling elements (5, 6) at least in some sections, wherein the union clamp has a clamp profile (11), which applies a clamping load onto the frustoconical expansions (10) when accommodating the coupling elements (5, 6) in the union clamp.

4. A system (1) according to claim 1, **characterized in that** the recess (9) for passing through the foils (7) is provided in the top part (13) of the union clamp.

5. A system (1) according to claim 1 or 4, **characterized in that** the bottom part (11), if the first coupling element (5) and the second coupling element (6) are accommodated in the union clamp, will abut only in a central section (17) the first coupling element (5) and the second coupling element (6).

6. A system (1) according to any of the claims 1 to 5, **characterized in that** the first coupling element (5) and the second coupling element (6) each have a sealing (18) circumferential to the orifice (4) and covered by the foil (7).

7. A system (1) according to claim 1, **characterized in that** das fixing element (15) in the region of the clamping element (14) comprises an eccentric (19) acting on the top part (13), wherein the eccentric (19) applies a pressure load onto the top part (13) if the fixing element (15) spans the top part (13) of the union clamp.

8. A system (1) according to any of the claims 1 to 7, **characterized in that** the first coupling element (5) and the second coupling element (6) comprise localization elements (20) that may be coupled to one another, which are configured to position the orifice (4) of the first fluid channel (2) essentially congruently with the orifice (4) of the second fluid channel (3) in the course of a coupling.

9. A system (1) according to any of the claims 1 to 8, **characterized in that** the foils (7) each comprise a pull-tab (21) folded over at a fringe region of the orifices (4), which may be passed through the recess (9) of the connecting element (8).

10. A method for providing a sterile fluid connection to a system (1) according to claim 1, **characterized by** the following steps:
- Arranging the orifice (4) of the first fluid channel (2) essentially congruently with the orifice (4) of the second fluid channel (3);
- Accommodating at least in some section the first coupling element (5) and the second coupling element (6) in the regions of the orifices (4) of the fluid channels (2, 3) by the connecting element (8);
- Passing the foils (7) closing the orifices (4) of the fluid channels (2, 3), which may be peeled off of the respective orifice (4), through the recess (9) in the connecting element (8);
- Producing the sterile fluid connection by simultaneous peeling off the foils (7) through the recess (9) of the connecting element (8),
- Spanning the top part (13) of the union nut with the fixing element (15),
- Locking the fixing element (15) in the position spanning the top part (13) of the union clamp by means of the locking element (16).

11. A method for providing a sterile fluid connection according to claim 10 to a system (1) according to the claims 1 and 4, **characterized in that** the step of passing the foils (7) closing the orifices (4) of the fluid channels (2, 3), which may be peeled off of the respective orifice (4), through the recess (9) in the connecting element (8) comprises covering the top part (13) of the union clamp, passing the foils (7) closing the orifice (4) of the first fluid channel (2) and of the second fluid channel (3) through the recess (9) in the top part (13) of the union clamp and locking the top part (13) at the bottom part (11) by means of the clamping element (14).

## Revendications

1. Système (1) destiné à fournir une liaison fluidique stérile entre un premier canal de fluide (2) et un second canal de fluide (3), comprenant un premier élément de couplage (5), disposé au niveau d'une embouchure (4) du premier canal de fluide (2), et un second élément de couplage (6), disposé au niveau d'une embouchure (4) du second canal de fluide (3), dans lequel le premier élément de couplage (5) et le second élément de couplage (6) comprennent chacun un film (7) qui obture l'embouchure (4) du canal de fluide respectif (2, 3) et qui peut être enlevé de l'embouchure (4),
dans lequel le système (1) comprend un élément de liaison (8), qui est séparé du premier élément de couplage (5) et du second élément de couplage (6), et qui est conçu de manière à recevoir au moins par sections le premier élément de couplage (5) et le second élément de couplage (6) au niveau des embouchures (4) des canaux de fluide (2, 3) et pour presser les unes contre les autres les embouchures (4) des canaux de fluide (2, 3) obturées par les films (7), l'élément de liaison (8) présentant un évidement (9) disposé sensiblement dans le plan des embouchures (4) des canaux de fluide (2, 3) afin de guider les films (7) à travers l'élément de liaison (8),
**caractérisé en ce que**
l'élément de liaison (8) est conçu sous la forme d'une pince à verrouillage et
la pince à verrouillage présente une partie inférieure (11), une partie supérieure (13) reliée à la partie inférieure (11) à l'aide d'une articulation formant charnière (12) et un élément de blocage (14) disposé au niveau d'un côté sensiblement opposé à l'articulation formant charnière (12) afin de bloquer la partie supérieure (13) sur la partie inférieure (11),
dans lequel la pince à verrouillage comprend un élément de fixation (15) relié de manière articulée à l'élément de blocage (14) et un élément de blocage (16) disposé au niveau de l'articulation formant charnière (13), l'élément de fixation (15) étant conçu de manière à recouvrir la partie supérieure (13) de la pince à verrouillage à partir de l'élément de blocage (14), et l'élément de blocage (16) est conçu de manière à bloquer l'élément de fixation (15) dans une position recouvrant la partie supérieure (13) de la pince à verrouillage.

2. Système (1) selon la revendication 1, **caractérisé en ce que** le premier élément de couplage (5) et le second élément de couplage (6) présentent un élargissement tronconique (10) au niveau des embouchures (4).

3. Système (1) selon la revendication 2, **caractérisé en ce que** la pince à verrouillage est conçue de manière à recevoir au moins par sections les élargissements tronconiques (10) des éléments de couplage (5, 6), la pince à verrouillage présentant un profil de blocage qui exerce une sollicitation de blocage sur les élargissements tronconiques (10) lors de la réception des éléments de couplage (5, 6) dans la pince à verrouillage.

4. Système (1) selon la revendication 1, **caractérisé en ce que** l'évidement (9) est prévu afin de guider les films (7) dans la partie supérieure (13) de la pince à verrouillage.

5. Système (1) selon une des revendications 1 ou 4, **caractérisé en ce que** la partie inférieure (11), lorsque le premier élément de couplage (5) et le second élément de couplage (6) sont reçus dans la pince à verrouillage, se situe uniquement dans une section centrale (17) au niveau du premier élément de couplage (5) et du second élément de couplage (6).

6. Système (1) selon une des revendications 1 à 5, **caractérisé en ce que** le premier élément de couplage (5) et le second élément de couplage (6) présentent chacun un joint d'étanchéité (18) circonférentiel à l'embouchure (4) et recouvert par le film (7).

7. Système (1) selon la revendication 1, **caractérisé en ce que** l'élément de fixation (15) comprend au niveau de l'élément de blocage (14) un excentrique (19) qui agit sur la partie supérieure (13), l'excentrique (19) exerçant une compression sur la partie supérieure (13) lorsque l'élément de fixation (15) recouvre la partie supérieure (13) de la pince à verrouillage.

8. Système (1) selon une des revendications 1 à 7, **caractérisé en ce que** le premier élément de couplage (5) et le second élément de couplage (6) comprennent des éléments de localisation (20) qui peuvent être couplés les uns avec les autres et qui sont conçus de manière à positionner, au cours d'un couplage, l'embouchure (4) du premier canal de fluide (2) de manière sensiblement superposable avec l'embouchure (4) du second canal de fluide (3).

9. Système (1) selon une des revendications 1 à 8, **caractérisé en ce que** les films (7) comprennent chacun une bride d'enlèvement (21) rabattue au niveau d'une zone de bordure des embouchures (4) et qui peut être guidée à travers l'évidement (9) de l'élément de liaison (8).

10. Procédé destiné à fournir une liaison fluidique stérile avec un système (1) selon la revendication 1, **caractérisé par** les étapes suivantes :
- disposition de l'embouchure (4) du premier canal de fluide (2) de manière sensiblement superposable avec l'embouchure (4) du second canal de fluide (3) ;
- réception au moins par sections du premier élément de couplage (5) et du second élément de couplage (6) au niveau des embouchures (4) des canaux de fluide (2, 3) à travers l'élément de liaison (8) ;
- guidage des films qui obturent les embouchures (4) des canaux de fluide (2, 3) et qui peuvent être enlevés de l'embouchure respective (4) à travers l'évidement (9) dans l'élément de liaison (8) ;
- fabrication de la liaison fluidique stérile en enlevant simultanément les films (7) à travers l'évidement (9) de l'élément de liaison (8),
- recouvrement de la partie supérieure (13) de l'écrou-raccord avec l'élément de fixation (15) ;
- blocage de l'élément de fixation (15) dans la position qui recouvre la partie supérieure (13) de la pince à verrouillage à l'aide de l'élément de blocage (16).

11. Procédé de fourniture d'une liaison fluidique stérile selon la revendication 10, avec un système (1) selon les revendications 1 et 4, **caractérisé en ce que** l'étape de guidage des films qui obturent les embouchures (4) des canaux de fluide (2, 3) et qui peuvent être enlevés de l'embouchure (4) respective à travers l'évidement (9) dans l'élément de liaison (8) comprend la fermeture de la partie supérieure (13) de la pince à verrouillage, le guidage des films (7) qui obturent l'embouchure (4) du premier canal de fluide (2) et du second canal de fluide (3) à travers l'évidement (9) dans la partie supérieure (13) de la pince à verrouillage, et le blocage de la partie supérieure (13) au niveau de la partie inférieure (11) à l'aide de l'élément de blocage (14).
